# EUROPEAN PATENT APPLICATION

(11) **EP 4 566 697 A1**
(43) Date of publication of application: **11.06.2025**
(21) Application number: 23850122.5
(22) Date of filing: 02.08.2023
(51) Int. Cl.: B01D 63/06

(54) **SEPARATION MEMBRANE MODULE**

(30) Priority: 02.08.2022 JP 2022123563
(71) Applicant: NGK Insulators, Ltd., Nagoya-shi, Aichi 467-8530 (JP)
(72) Inventor: IIDA, Kazuki, Nagoya-shi, Aichi 467-8530 (JP); SHIBAGAKI, Yukinari, Nagoya-shi, Aichi 467-8530 (JP); NAKAGAWA, Kosuke, Nagoya-shi, Aichi 467-8530 (JP); MAEHARA, Sota, Nagoya-shi, Aichi 467-8530 (JP); KAN, Hirofumi, Nagoya-shi, Aichi 467-8530 (JP)
(74) Representative: Müller-Boré & Partner Patentanwälte PartG mbB
(86) International application number: PCT/JP2023/028295
(87) International publication number: WO 2024/029568

(57) **Abstract**

A separation membrane module (1) includes a tubular housing (20), a columnar reactor (10) housed in the housing (20) and extending in a longitudinal direction, and an annular first flange (30) surrounding a first end portion (10a) of the reactor (10). A first end surface (F2) of the reactor (10) is located outward of an end surface (K1) of the first flange (30) in the longitudinal direction.

## Description

### TECHNICAL FIELD

The present invention relates to a separation membrane module.

### BACKGROUND ART

Separation membrane modules including a housing and a columnar membrane structure housed in the housing are conventionally known. Examples of the membrane structure include a separation filter (see, for example, Patent Literature 1) configured to separate a predetermined component from a fluid mixture with use of a separation membrane, and a reactor (see, for example, Patent Literature 2) configured to separate a product generated through a conversion reaction for converting a source gas into a liquid fuel with use of a separation membrane.

### CITATION LIST

### Patent Literature

Patent Literature 1: WO 2018/180095
Patent Literature 2: JP 2018-008940A

### SUMMARY

### TECHNICAL PROBLEM

Incidentally, a permeate-side space for collecting a component that has passed through a separation membrane needs to be provided between the membrane structure and the housing, and therefore, it is necessary to attach a flange to an end portion of the membrane structure.

On the other hand, a longitudinal dimension of the membrane structure may vary due to variations that occur in the manufacture of the membrane structure, and accordingly, the longitudinal dimension of the membrane structure may be larger than a designed value. In such a case, it is very troublesome to increase a longitudinal dimension of the housing in order to house the membrane structure.

An object of the present invention is to provide a separation membrane module in which a membrane structure can be housed irrespective of the longitudinal dimension of the membrane structure.

### SOLUTION TO PROBLEM

A separation membrane module according to a first aspect includes a tubular housing, a columnar membrane structure housed in the housing and extending in a longitudinal direction, and an annular first flange surrounding a first end portion of the membrane structure. A first end surface of the membrane structure is located outward of an end surface of the first flange in the longitudinal direction.

In a separation membrane module according to a second aspect, which is the separation membrane module according to the first aspect, the housing includes a first opening having a center on an axis of the membrane structure, and an outer circumferential surface of the membrane structure is located inward of an inner circumferential surface of the first opening in a radial direction perpendicular to the longitudinal direction.

In a separation membrane module according to a third aspect, which is the separation membrane module according to the first or the second aspect, a portion of the membrane structure is inserted into the first opening.

A separation membrane module according to a fourth aspect is the separation membrane module according to any of the first through third aspects, further including a first joining material interposed between the membrane structure and the first flange. The first joining material is spaced apart from the housing.

In a separation membrane module according to a fifth aspect, which is the separation membrane module according to any of the first through fourth aspects, the first flange is constituted by a ceramic material.

A separation membrane module according to a sixth aspect is the separation membrane module according to any of the first through fifth aspects, further including an annular second flange surrounding a second end portion of the membrane structure. A second end surface of the membrane structure is located outward of an end surface of the second flange in the longitudinal direction.

In a separation membrane module according to a seventh aspect, which is the separation membrane module according to the sixth aspect, the housing includes a second opening having a center on an axis of the membrane structure, and an outer circumferential surface of the membrane structure is located inward of an inner circumferential surface of the second opening in the radial direction.

In a separation membrane module according to an eighth aspect, which is the separation membrane module according to the sixth or the seventh aspect, a portion of the membrane structure is inserted into the second opening.

A separation membrane module according to a ninth aspect is the separation membrane module according to any of the sixth through eighth aspects, further including a second joining material interposed between the membrane structure and the second flange. The second joining material is spaced apart from the housing.

In a separation membrane module according to a tenth aspect, which is the separation membrane module according to any of the sixth through ninth aspects, the second flange is constituted by a ceramic material.

In a separation membrane module according to an eleventh aspect, which is the separation membrane module according to any of the first through tenth aspects, the membrane structure is a reactor.

In a separation membrane module according to a twelfth aspect, which is the separation membrane module according to any of the first through tenth aspects, the membrane structure is a separation filter.

### ADVANTAGEOUS EFFECTS

According to the present invention, it is possible to provide a separation membrane module in which a membrane structure can be housed irrespective of the longitudinal dimension of the membrane structure.

### BRIEF DESCRIPTION OF DRAWINGS

FIG. 1 is a cross-sectional view of a separation membrane module according to an embodiment.
FIG. 2 is a partial enlarged view of FIG. 1.
FIG. 3 is a partial enlarged view of FIG. 1.
FIG. 4 is a cross-sectional view of a separation membrane module according to Variation 2.
FIG. 5 is a cross-sectional view taken along a line A-A shown in FIG. 4.
FIG. 6 is a cross-sectional view taken along a line B-B shown in FIG. 4.
FIG. 7 is a cross-sectional view of a separation membrane module according to Variation 5.
FIG. 8 is a cross-sectional view of a separation membrane module according to Variation 7.
FIG. 9 is a cross-sectional view of a separation membrane module according to Variation 7.
FIG. 10 is a cross-sectional view of a separation membrane module according to Variation 8.
FIG. 11 is a cross-sectional view of a separation membrane module according to Variation 8.
FIG. 12 is a cross-sectional view of a separation membrane module according to Variation 8.
FIG. 13 is a cross-sectional view of a separation membrane module according to Variation 9.
FIG. 14 is a cross-sectional view of a separation membrane module according to Variation 9.
FIG. 15 is a cross-sectional view of a separation membrane module according to Variation 10.
FIG. 16 is a cross-sectional view of a separation membrane module according to Variation 10.
FIG. 17 is a cross-sectional view of a separation membrane module according to Variation 10.

### DESCRIPTION OF EMBODIMENTS

### (Separation membrane module 1)

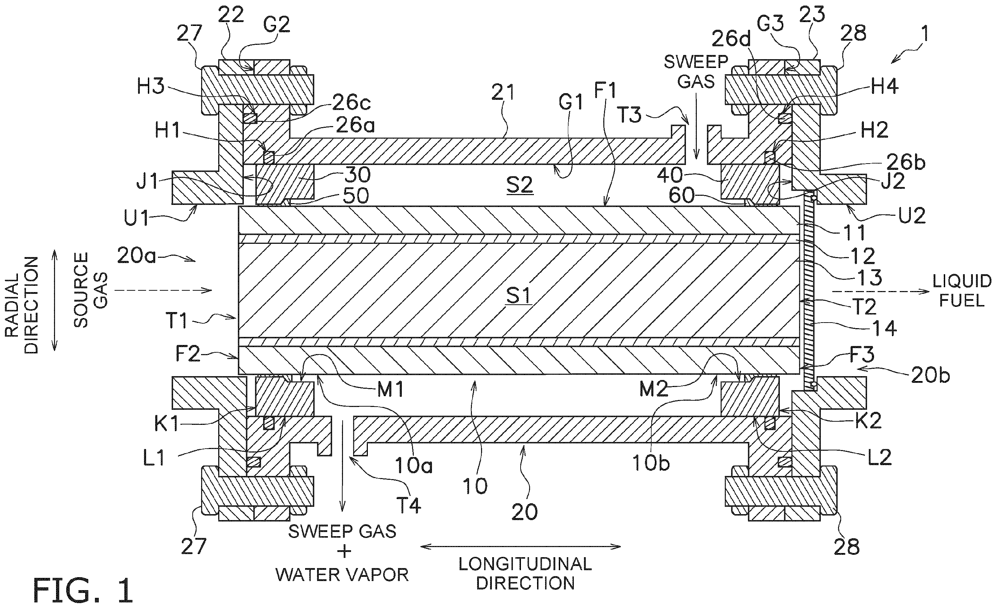

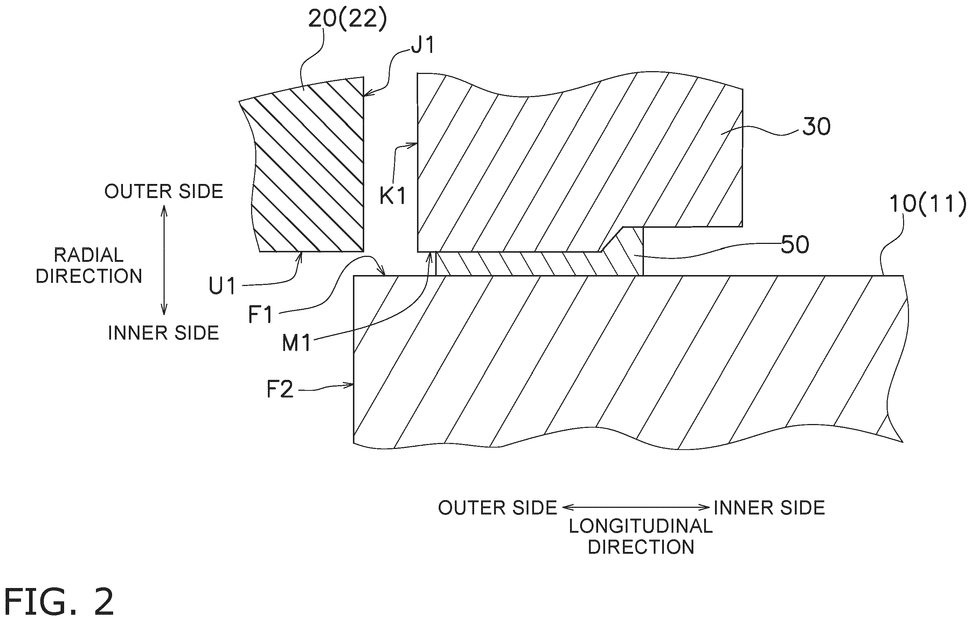

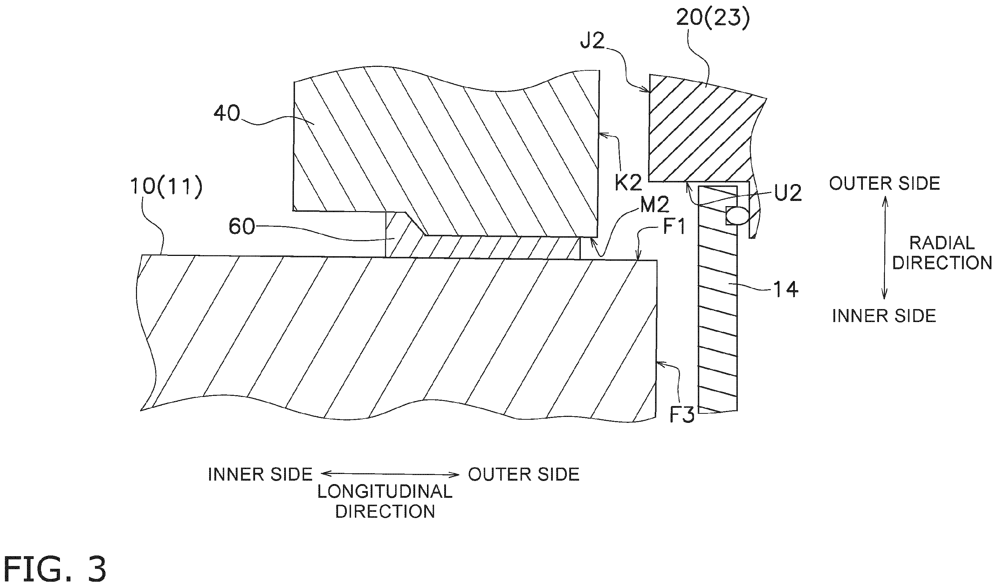

The following describes a separation membrane module 1 according to an embodiment. FIG. 1 is a schematic cross-sectional view showing a configuration of the separation membrane module 1. FIGS. 2 and 3 are partial enlarged views of FIG. 1.

As shown in FIG. 1, the separation membrane module 1 includes a reactor 10, a housing 20, a first flange 30, a second flange 40, a first joining material 50, and a second joining material 60. The reactor 10 is an example of a "membrane structure" according to the present invention.

### [Reactor 10]

The reactor 10 is housed in the housing 20. The reactor 10 is formed into a columnar shape extending in a longitudinal direction. The external shape of the reactor 10 is not particularly limited, and may be a round column shape, an elliptical column shape, or a polygonal column shape, for example.

The reactor 10 is a so-called membrane reactor for converting a source gas into a liquid fuel. The source gas contains at least hydrogen and carbon dioxide. The source gas may also contain carbon monoxide. The source gas may be a so-called synthesis gas (syngas). The liquid fuel is a fuel that is in a liquid state at normal temperature and pressure or a fuel that can be liquefied at normal temperature in a pressurized state. Examples of fuels that are in the liquid state at normal temperature and pressure include methanol, ethanol, a liquid fuel represented by CₙH₂₍ₘ₋₂ₙ₎ (m is an integer smaller than 90, and n is an integer smaller than 30), and a mixture of these. Examples of fuels that can be liquefied at normal temperature in a pressurized state include propane, butane, and a mixture of these.

For example, a reaction formula (1) for the synthesis of methanol through catalytic hydrogenation of a source gas containing hydrogen and carbon dioxide in the presence of a catalyst is expressed as follows.

CO₂+3H₂ ⇔ CH₃OH+H₂O (1)

The above reaction is an equilibrium reaction, and the reactor 10 can shift the reaction equilibrium to the product side by separating water vapor, which is a product of the conversion reaction. In order to increase the conversion efficiency and the reaction rate, the conversion reaction is preferably carried out at a high temperature and a high pressure (e.g., 180°C or higher and 2 MPa or higher). The liquid fuel is in a gaseous state when it is synthesized, and remains in the gaseous state at least until the liquid fuel flows out of the reactor 10. It is preferable that the reactor 10 has heat resistance and pressure resistance appropriate for synthesis conditions of the desired liquid fuel.

The reactor 10 according to the present embodiment is a so-called tubular type. As shown in FIG. 1, the reactor 10 includes an outer circumferential surface F1, a first end surface F2, and a second end surface F3. The outer circumferential surface F1 is a side surface of the columnar reactor 10. The outer circumferential surface F1 is continuous with the first end surface F2 and the second end surface F3. The first end surface F2 is an end surface of the columnar reactor 10. A first opening T1 is formed in the first end surface F2. The source gas flows into the reactor 10 via the first opening T1. The second end surface F3 is another end surface of the columnar reactor 10. A second opening T2 is formed in the second end surface F3. The liquid fuel flows out of the reactor 10 via the second opening T2.

The reactor 10 includes a first end portion 10a and a second end portion 10b. The first end portion 10a is an end portion of the reactor 10 in the longitudinal direction. The first end portion 10a includes the first end surface F2 described above. The second end portion 10b is another end portion of the reactor 10 in the longitudinal direction. The second end portion 10b includes the second end surface F3 described above.

Here, the reactor 10 is constituted by a porous support 11, a separation membrane 12, a catalyst 13, and a catalyst stopper 14.

The porous support 11 is formed into a tubular shape extending in the longitudinal direction. The porous support 11 is constituted by a porous material. A ceramic material, a metallic material, a resin material, a composite member of these, or the like can be used as the porous material, and a ceramic material is particularly favorable. Alumina (Al₂O₃), titania (TiO₂), mullite (Al₂O₃·SiO₂), potsherd, cordierite (Mg₂Al₄Si₅O₁₈), a composite material including two or more of these, or the like can be used as an aggregate for the ceramic material. Alumina is favorable in view of availability, clay stability, and corrosion resistance. At least one of titania, mullite, easily sinterable alumina, silica, glass frit, a clay mineral, and easily sinterable cordierite can be used as an inorganic binder for the ceramic material. However, the ceramic material need not necessarily contain an inorganic binder.

The average pore diameter of the porous support 11 can be set to 5 µm or more and 25 µm or less. The average pore diameter of the porous support 11 can be measured using a mercury intrusion method. The porosity of the porous support 11 can be set to 25% or more and 50% or less. The average particle diameter of the porous material can be set to 1 µm or more and 100 µm or less. The average particle diameter refers to the arithmetic mean of the maximum diameters of 30 particles to be measured (randomly selected), as measured by cross-sectional microstructural observation using a scanning electron microscope (SEM).

The separation membrane 12 is supported by the porous support 11. The separation membrane 12 is formed into a tubular shape extending in the longitudinal direction. The inner side of the separation membrane 12 is a non-permeate-side space S1 to which the source gas is supplied. The non-permeate-side space S1 is a space between the first opening T1 and the second opening T2. In the present embodiment, the separation membrane 12 is arranged on the inner surface of the porous support 11, but the separation membrane 12 may be arranged on the outer surface of the porous support 11.

The separation membrane 12 allows permeation of water vapor, which is a product generated through the conversion reaction for converting the source gas into the liquid fuel. Thus, the reaction equilibrium of the above formula (1) can be shifted to the product side by utilizing an equilibrium shift effect.

It is preferable that the separation membrane 12 has a water vapor permeability coefficient of 100 nmol/(s·Pa·m²) or more. The water vapor permeability coefficient can be obtained using a known method (see Ind. Eng. Chem. Res., 40, 163-175 (2001)).

It is preferable that the separation membrane 12 has a separation coefficient of 100 or more. The larger the separation coefficient is, the separation membrane 12 allows permeation of more water vapor and less components (hydrogen, carbon dioxide, the liquid fuel, etc.) other than water vapor. The separation coefficient can be obtained using a known method (see FIG. 1 in "Separation and Purification Technology 239 (2020) 116533").

The separation membrane 12 may be an inorganic membrane. Inorganic membranes have heat resistance, pressure resistance, and water vapor resistance and thus are preferable. Examples of inorganic membranes include zeolite membranes, silica membranes, alumina membranes, and composite membranes thereof. In particular, LTA-type zeolite membranes in which a mole ratio (Si/Al) between silicon element (Si) and aluminum element (Al) is 1.0 or more and 3.0 or less have excellent water vapor permeability and thus are favorable.

The catalyst 13 is arranged on the inner side of the separation membrane 12, i.e., in the non-permeate-side space S1. It is preferable that the non-permeate-side space S1 is filled with the catalyst 13, but the catalyst 13 may also be arranged in such a manner as to form a layer or islands on the surface of the separation membrane 12. The catalyst 13 promotes the conversion reaction for converting the source gas into the liquid fuel shown in the above formula (1).

A known catalyst suitable for the conversion reaction for converting the source gas into the liquid fuel can be used as the catalyst 13. Examples of the catalyst 13 include metal catalysts (copper, palladium, etc.), oxide catalysts (zinc oxide, zirconia, gallium oxide, etc.), and composites of these (copper-zinc oxide, copper-zinc oxide-alumina, copper-zinc oxide-chromium oxide-alumina, copper-cobalt-titania, and these catalysts modified with palladium, etc.).

The catalyst stopper 14 is arranged in such a manner as to cover the second opening T2 formed in the second end surface F3. The catalyst stopper 14 prevents the catalyst 13 from leaking from the second opening T2. The catalyst stopper 14 is configured so as not to prevent the liquid fuel from flowing out, while preventing leakage of the catalyst 13. A net-like member, a plate having a hole, or the like can be used as the catalyst stopper 14. In the present embodiment, the catalyst stopper 14 is attached to the housing 20, but the catalyst stopper 14 may also be attached to the second end surface F3 of the reactor 10.

However, in a case where leakage of the catalyst 13 is unlikely to occur (for example, in the case where the catalyst 13 is arranged in such a manner as to form a layer or islands on the surface of the separation membrane 12), the reactor 10 need not be provided with the catalyst stopper 14.

In the reactor 10, the source gas supplied to the non-permeate-side space S1 is converted into the liquid fuel due to the action of the catalyst 13, and water vapor, which is a product generated through the conversion reaction, is separated by passing through the separation membrane 12 and the porous support 11 into a permeate-side space S2, which will be described later.

### [Housing 20]

The housing 20 is formed into a tubular shape as a whole. The housing 20 accommodates the reactor 10. The housing 20 has a structure that can endure the conversion reaction carried out at a high temperature and a high pressure (e.g., 180°C or higher and 2 MPa or higher). In a case where the source gas and/or sweep gas contains hydrogen, it is preferable that the housing 20 is constituted by a material that is resistant to hydrogen embrittlement. The housing 20 can be constituted mainly by a metallic material (stainless steel or the like).

As shown in FIG. 1, the housing 20 is constituted by a tube main body 21, a first end plate 22, and a second end plate 23.

The tube main body 21 is formed into a tubular shape extending in the longitudinal direction. Both end portions of the tube main body 21 are formed like flanges by increasing the diameter.

The tube main body 21 has an inner circumferential surface G1, a first end surface G2, a second end surface G3, a sweep gas inlet T3, and a sweep gas outlet T4.

The inner circumferential surface G1 faces the outer circumferential surface F1 of the reactor 10 and is spaced apart from the outer circumferential surface F1. The space between the inner circumferential surface G1 and the outer circumferential surface F1 is the permeate-side space S2 for collecting water vapor, which is a product generated through the conversion reaction.

An annular first recess H1 is formed at an end of the inner circumferential surface G1. An annular first elastic member 26a is arranged in the first recess H1. The first elastic member 26a may be an O-ring made of expanded graphite or rubber, for example. The first elastic member 26a is in intimate contact with the first flange 30, which will be described later. Thus, a seal is formed between the tube main body 21 and the first flange 30.

An annular second recess H2 is formed at another end of the inner circumferential surface G1. An annular second elastic member 26b is arranged in the second recess H2. The second elastic member 26b may be an O-ring made of expanded graphite or rubber, for example. The second elastic member 26b is intimate contact with the second flange 40, which will be described later. Thus, a seal is formed between the tube main body 21 and the second flange 40.

An annular first recess H3 is formed in the first end surface G2. A third elastic member 26c is arranged in the first recess H3. The third elastic member 26c may be an O-ring made of expanded graphite or rubber, for example. The third elastic member 26c is in intimate contact with the first end plate 22. Thus, a seal is formed between the tube main body 21 and the first end plate 22.

An annular second recess H4 is formed in the second end surface G3. A fourth elastic member 26d is arranged in the second recess H4. The fourth elastic member 26d may be an O-ring made of expanded graphite or rubber, for example. The fourth elastic member 26d is in intimate contact with the second end plate 23. Thus, a seal is formed between the tube main body 21 and the second end plate 23.

Each of the sweep gas inlet T3 and the sweep gas outlet T4 is continuous with the permeate-side space S2. Sweep gas is supplied via the sweep gas inlet T3 to the permeate-side space S2. In the permeate-side space S2, the sweep gas takes in water vapor and absorbs reaction heat generated through the conversion reaction. The sweep gas is discharged from the sweep gas outlet T4 together with water vapor. Hydrogen and/or carbon dioxide can be used as the sweep gas. Alternatively, inert gas (e.g., nitrogen), air, or the like may be used as the sweep gas. In the present embodiment, the sweep gas inlet T3 and the sweep gas outlet T4 are diagonally opposite to each other in a cross-sectional view, but the positions of the sweep gas inlet T3 and the sweep gas outlet T4 can be suitably changed.

The first end plate 22 is an annular plate member. The first end plate 22 has a first opening 20a whose center is on the axis of the reactor 10. The shape of the first opening 20a is not particularly limited, and may be a circular shape or a polygonal shape, for example. A portion of the first end plate 22 surrounding the first opening 20a is formed like a flange by increasing the diameter.

As shown in FIGS. 1 and 2, the first end plate 22 has a facing surface J1 and an inner circumferential surface U1.

The facing surface J1 faces an end surface K1 of the first flange 30, which will be described later. In the present embodiment, the facing surface J1 is spaced apart from the end surface K1 of the first flange 30. The facing surface J1 may abut against at least a portion of the end surface K1 of the first flange 30. The facing surface J1 abuts against the first end surface G2 of the tube main body 21. An annular third recess H5 is formed in the first facing surface J1.

The inner circumferential surface U1 is continuous with the facing surface J1. The inner circumferential surface U1 forms a tubular shape. The inner circumferential surface U1 is located outward of the outer circumferential surface F1 of the reactor 10 in a radial direction (direction perpendicular to the longitudinal direction). In the present embodiment, a portion of the reactor 10 (specifically, an end of the first end portion 10a) is inserted into the first opening 20a. Accordingly, the inner circumferential surface U1 faces a portion of the outer circumferential surface F1 of the reactor 10 in the radial direction.

Note that "outward in the radial direction" means the side away from the axis of the reactor 10 in the radial direction, and "inward in the radial direction" means the side closer to the axis of the reactor 10 in the radial direction.

The first end plate 22 is connected to the tube main body 21 with use of a plurality of fixing members 27. The fixing members 27 are constituted by bolts and nuts, for example. The first end plate 22 is in intimate contact with the third elastic member 26c.

The second end plate 23 is an annular plate member. The second end plate 23 has a second opening 20b whose center is on the axis of the reactor 10. The shape of the second opening 20b is not particularly limited, and may be a circular shape or a polygonal shape, for example. A portion of the second end plate 23 surrounding the second opening 20b is formed like a flange by increasing the diameter.

As shown in FIGS. 1 and 3, the second end plate 23 has a facing surface J2 and an inner circumferential surface U2.

The facing surface J2 faces an end surface K2 of the second flange 40, which will be described later. In the present embodiment, the second facing surface J2 is spaced apart from the end surface K2 of the second flange 40. The facing surface J2 may abut against at least a portion of the end surface K2 of the second flange 40. The facing surface J2 abuts against the second end surface G3 of the tube main body 21. An annular fourth recess H6 is formed in the second facing surface J2.

The inner circumferential surface U2 is continuous with the facing surface J2. The inner circumferential surface U2 forms a tubular shape. The inner circumferential surface U2 is located outward of the outer circumferential surface F1 of the reactor 10 in the radial direction. In the present embodiment, a portion of the reactor 10 (specifically, an end of the second end portion 10b) is inserted into the second opening 20b. Accordingly, the inner circumferential surface U2 faces a portion of the outer circumferential surface F1 of the reactor 10 in the radial direction.

The second end plate 23 is connected to the tube main body 21 with use of a plurality of fixing members 28. The fixing members 28 are constituted by bolts and nuts, for example. The second end plate 23 is in intimate contact with the fourth elastic member 26d.

### [First flange 30]

The first flange 30 is attached to the reactor 10. The first flange 30 is joined to the reactor 10 by the first joining material 50. The first flange 30 serves as a spacer for forming the permeate-side space S2 between the reactor 10 and the tube main body 21. The first flange 30 is formed into an annular shape. The first flange 30 surrounds the first end portion 10a of the reactor 10. The first flange 30 is fitted into an end portion of the tube main body 21. The first flange 30 supports the first end portion 10a of the reactor 10 at a position spaced apart from the tube main body 21. Thus, the permeate-side space S2 is formed between the reactor 10 and the tube main body 21.

As shown in FIGS. 1 and 2, the first flange 30 has the end surface K1, an outer circumferential surface L1, and an inner circumferential surface M1.

The end surface K1 is an outer surface of the first flange 30 in the longitudinal direction. In the longitudinal direction, the end surface K1 is located inward of the first end surface F2 of the reactor 10. In other words, the first end surface F2 of the reactor 10 is located outward of the end surface K1 of the first flange 30 in the longitudinal direction. Therefore, the first flange 30 does not interfere with the reactor 10 in the longitudinal direction, and accordingly, it is possible to freely set the position of the first flange 30 relative to the reactor 10 in the longitudinal direction. Therefore, even when the longitudinal dimension of the reactor 10 is larger than a designed value, the reactor 10 can be housed in the housing 20 irrespective of the longitudinal dimension of the reactor 10. Consequently, the cost of the separation membrane module 1 can be reduced because there is no need to prepare multiple types of housings 20 having different longitudinal dimensions.

Note that "outward in the longitudinal direction" means the side away from the center of the reactor 10 in the longitudinal direction, and "inward in the longitudinal direction" means the side closer to the center of the reactor 10 in the longitudinal direction.

Also, the end surface K1 faces the facing surface J1 of the first end plate 22. At least a portion of the end surface K1 may abut against the facing surface J1. In the present embodiment, the end surface K1 is a planar surface. The outer circumferential surface L1 is a radially outer surface of the first flange 30. The outer circumferential surface L1 faces the inner circumferential surface G1 of the tube main body 21 and is in intimate contact with the first elastic member 26a. The outer circumferential surface L1 may abut against the inner circumferential surface G1. The inner circumferential surface M1 is on the side opposite to the outer circumferential surface L1. The inner circumferential surface M1 faces the outer circumferential surface F1 of the reactor 10 via the first joining material 50.

The first flange 30 is constituted by a dense ceramic material. Examples of the ceramic material include alumina, zirconia, silicon carbide, aluminum nitride, cordierite, and a composite material including two or more of these. The first flange 30 needs to be air-tight and liquid-tight. Accordingly, the porosity of the first flange 30 is preferably 10.0% or less, and more preferably 5.0% or less.

### [Second flange 40]

The second flange 40 is attached to the reactor 10. The second flange 40 is joined to the reactor 10 by the second joining material 60. The second flange 40 is arranged on the side opposite to the first flange 30. The second flange 40 serves as a spacer for forming the permeate-side space S2 between the reactor 10 and the tube main body 21. The second flange 40 is formed into an annular shape. The second flange 40 surrounds the second end portion 10b of the reactor 10. The second flange 40 is fitted into another end portion of the tube main body 21. The second flange 40 supports the second end portion 10b of the reactor 10 at a position spaced apart from the tube main body 21. Thus, the permeate-side space S2 is formed between the reactor 10 and the tube main body 21.

As shown in FIGS. 1 and 2, the second flange 40 has the end surface K2, an outer circumferential surface L2, and an inner circumferential surface M2.

The end surface K2 is an outer surface of the second flange 40 in the longitudinal direction. In the longitudinal direction, the end surface K2 is located inward of the second end surface F3 of the reactor 10. In other words, the second end surface F3 of the reactor 10 is located outward of the end surface K2 of the second flange 40 in the longitudinal direction. Therefore, the second flange 40 does not interfere with the reactor 10 in the longitudinal direction, and accordingly, it is possible to freely set the position of the first flange 30 relative to the reactor 10 in the longitudinal direction. Therefore, even when the longitudinal dimension of the reactor 10 is larger than the designed value, the reactor 10 can be housed in the housing 20 irrespective of the longitudinal dimension of the reactor 10. Consequently, the cost of the separation membrane module 1 can be reduced because there is no need to prepare multiple types of housings 20 having different longitudinal dimensions.

Also, the end surface K2 faces the second facing surface J2 of the second end plate 23. At least a portion of the end surface K2 may abut against the second facing surface J2. In the present embodiment, the end surface K2 is a planar surface. The outer circumferential surface L2 is a radially outer surface of the second flange 40. The outer circumferential surface L2 faces the inner circumferential surface G1 of the tube main body 21 and is in intimate contact with the second elastic member 26b. The outer circumferential surface L2 may abut against the inner circumferential surface G1. The inner circumferential surface M2 is on the side opposite to the outer circumferential surface L2. The inner circumferential surface M2 faces the outer circumferential surface F1 of the reactor 10 via the second joining material 60.

The second flange 40 is constituted by a dense ceramic material. Examples of the ceramic material include alumina, zirconia, silicon carbide, aluminum nitride, cordierite, and a composite material including two or more of these. The second flange 40 needs to be air-tight and liquid-tight. Accordingly, the porosity of the second flange 40 is preferably 10.0% or less, and more preferably 5.0% or less.

### [First joining material 50]

The first joining material 50 is interposed between the first flange 30 and the reactor 10. The first joining material 50 joins the first flange 30 to the reactor 10. The first joining material 50 is arranged in at least a portion of a gap between the first flange 30 and the reactor 10.

It is preferable that the first joining material 50 is spaced apart from the housing 20 (specifically, the first end plate 22). In this case, the first joining material 50 can be kept from being damaged by interfering with the housing 20.

Note that a joined body constituted by the first flange 30 and the reactor 10 joined together by the first joining material 50 corresponds to a "separation membrane assembly" according to the present invention.

Crystallized glass, amorphous glass, a brazing material, ceramics, or the like can be used as the first joining material 50, and crystallized glass is particularly preferable in view of heat resistance and pressure resistance.

As the crystallized glass, it is possible to use SiO₂-B₂O₃-based, SiO₂-CaO-based, SiO₂-Al₂O₃-based, SiO₂-MgO-based, SiO₂-ZnO-BaO-based, SiO₂-B₂O₃-CaO-based, SiO₂-MgO-CaO-based, SiO₂-Al₂O₃-B₂O₃-based, or SiO₂-MgO-Al₂O₃-based crystallized glass, for example. Note that "crystallized glass" referred to in the present specification means glass in which a percentage of "the volume of a crystal phase" to the total volume (i.e., crystallinity) is 60% or more and a percentage of "the volume of an amorphous phase and impurities" to the whole volume is less than 40%.

### [Second joining material 60]

The second joining material 60 is interposed between the second flange 40 and the reactor 10. The second joining material 60 joins the second flange 40 to the reactor 10. The second joining material 60 is arranged in at least a portion of a gap between the second flange 40 and the reactor 10.

It is preferable that the second joining material 60 is spaced apart from the housing 20 (specifically, the second end plate 23). In this case, the second joining material 60 can be kept from being damaged by interfering with the housing 20.

Note that a joined body constituted by the second flange 40 and the reactor 10 joined together by the second joining material 60 corresponds to the "separation membrane assembly" according to the present invention.

Crystallized glass, amorphous glass, a brazing material, ceramics, or the like can be used as the second joining material 60, and crystallized glass is particularly preferable in view of heat resistance and pressure resistance.

### [Assembly of separation membrane module 1]

Steps for assembling the separation membrane module 1 include a step of producing a reactor assembly by joining the first and second flanges 30 and 40 to the reactor 10, and a step of housing the reactor assembly in the housing 20.

The step of producing a reactor assembly includes a first step of forming green bodies of the joining materials, a second step of attaching the flanges, and a third step of heating the green bodies of the joining materials. In the first step, a green body of the first joining material 50 is formed on the first end portion 10a of the reactor 10, and a green body of the second joining material 60 is formed on the second end portion 10b of the reactor 10. In the second step, the first flange 30 is attached in such a manner as to surround the green body of the first joining material 50, and the second flange 40 is attached in such a manner as to surround the green body of the second joining material 60. In the third step, the green bodies of the first and second joining materials 50 and 60 are heated to cause crystal growth or melting, and thereafter cooled to room temperature, and thus the first and second joining materials 50 and 60 are formed. Through the above steps, the reactor assembly in which the first and second flanges 30 and 40 are joined to the reactor 10 via the first and second joining materials 50 and 60 is completed.

Next, the step of housing the reactor assembly includes a fourth step of inserting the reactor assembly, a fifth step of attaching the elastic members, and a sixth step of connecting the end plates. In the fourth step, after the reactor assembly is inserted into the tube main body 21, positioning of both ends of the reactor assembly is performed. In the fifth step, the first through fourth elastic members 26a to 26d are fitted into the first through fourth recesses H1 to H4 of the tube main body 21. In the sixth step, the first end plate 22 is connected to the tube main body 21 with use of the fixing members 27, and the second end plate 23 is connected to the tube main body 21 with use of the fixing members 28. Thus, the separation membrane module 1 in which the reactor assembly is housed in the housing 20 is completed.

In the second step, the first and second flanges 30 and 40 can be attached to positions at which the first and second flanges 30 and 40 do not interfere with the first and second end plates 22 and 23 because positions of the first and second flanges 30 and 40 in the longitudinal direction relative to the reactor 10 can be freely set as described above. It is sufficient that a distance between the end surface K1 of the first flange 30 and the end surface K2 of the second flange 40 in the longitudinal direction is not longer than a distance between the facing surface J1 of the first end plate 22 and the facing surface J2 of the second end plate 23 in the longitudinal direction. **In** this case, the housing 20 does not interfere with the reactor 10 and the first and second flanges 30 and 40 in the fourth step, and accordingly, there is no need to prepare multiple types of housings 20 having different longitudinal dimensions.

### [Variations of embodiment]

Although an embodiment of the present invention has been described, the present invention is not limited to the above embodiment, and various changes can be made within a scope not departing from the gist of the present invention.

### [Variation 1]

**In** the above embodiment, a case where the reactor 10 is used as the membrane structure is described, but a separation filter may also be used as the membrane structure. The separation filter has a configuration similar to that of the reactor 10, except that a separation membrane that allows permeation of a desired component contained in a fluid mixture is used instead of the separation membrane 12 that allows permeation of water vapor, and the separation filter does not include the catalyst 13. In the case where the separation filter is used as the membrane structure as well, use of the separation filter under high-temperature and high-pressure conditions is assumed in the present invention.

Note that in the case where the separation filter is used as the membrane structure, reaction heat is not generated and the need to control the temperature is low. Accordingly, a component that has passed through the separation membrane may be discharged from the sweep gas outlet T4 by making the pressure on the sweep gas outlet T4 side lower than the pressure on the sweep gas inlet T3 side.

### [Variation 2]

In the above embodiment, the tubular type reactor 10 is described as an example of the membrane structure, but it is also possible to use a monolith type reactor. Also, in the case where a separation filter is used as the membrane structure, either a tubular type separation filter or a monolith type separation filter may be used. The monolith type is a concept that encompasses a honeycomb type and means a shape that includes a plurality of cells extending through the membrane structure in the longitudinal direction.

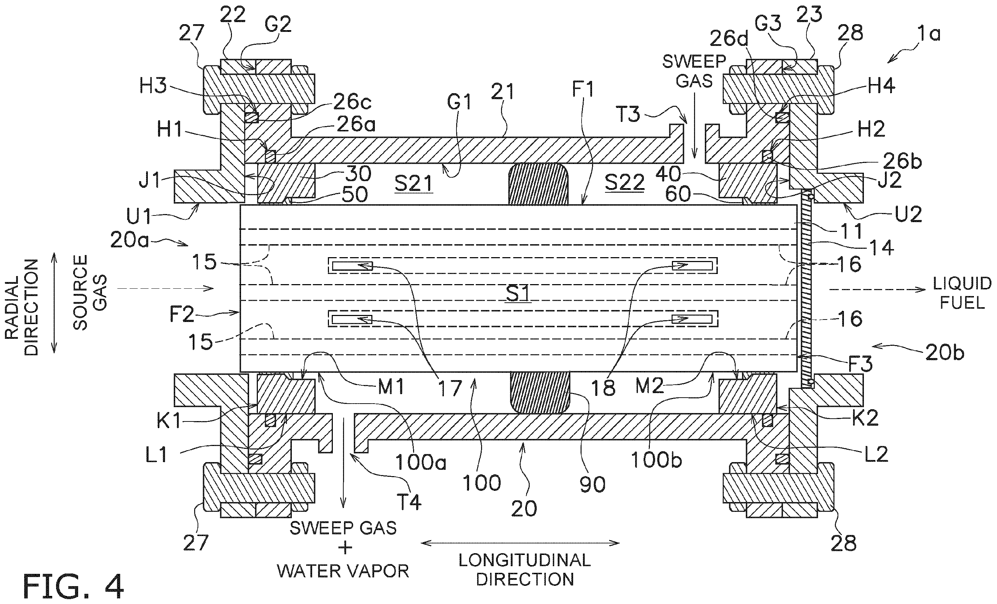

FIG. 4 is a schematic cross-sectional view showing a configuration of a separation membrane module 1a that includes a monolith type reactor 100. Note that only the reactor 100 is shown in a side view in FIG. 4.

The separation membrane module 1a is the same as the separation membrane module 1 according to the above embodiment, except that the separation membrane module 1a further includes a flow stopper 90, and includes the reactor 100 instead of the reactor 10. Note that the catalyst stopper 14 is optionally provided in the reactor 100 as well.

The flow stopper 90 is formed into an annular shape. The flow stopper 90 is arranged between the reactor 100 and the housing 20. The flow stopper 90 partitions the space between the reactor 100 and the housing 20 into a first permeate-side space S21 and a second permeate-side space S22. The flow stopper 90 suppresses a direct flow of sweep gas between the first permeate-side space S21 and the second permeate-side space S22. The flow stopper 90 is only required to be capable of suppressing a direct flow of sweep gas between these spaces, and need not serve as a seal between the reactor 100 and the housing 20. The flow stopper 90 can be made of expanded graphite, rubber, resin, metal, or the like, for example.

The reactor 100 includes a plurality of first flow paths 15, a plurality of second flow paths 16, first slits 17, and second slits 18.

The first flow paths 15 extend through the reactor 100 in the longitudinal direction. The first flow paths 15 are open in the first and second end surfaces F2 and F3. The separation membrane 12 described above is formed on the inner surface of each first flow path 15. The inner side of the separation membrane 12 is the non-permeate-side space S1. The catalyst 13 described above is arranged in the non-permeate-side space S1.

The second flow paths 16 are provided inside the reactor 100. The second flow paths 16 extend in the longitudinal direction. The second flow paths 16 are closed at the first and second end surfaces F2 and F3.

The first slits 17 are formed in a first end portion 100a of the reactor 100. The first slits 17 extend through each second flow path 16 in the radial direction and are open in the outer circumferential surface F1. Accordingly, the first slits 17 communicate with each second flow path 16 and the first permeate-side space S21.

The second slits 18 are formed in a second end portion 100b of the reactor 100. The second slits 18 extend through each second flow path 16 in the radial direction and are open in the outer circumferential surface F1. Accordingly, the second slits 18 communicate with each second flow path 16 and the second permeate-side space S22.

When the source gas is supplied to the first flow paths 15, the source gas is converted into the liquid fuel due to the action of the catalyst 13, and water vapor, which is a product of the conversion reaction, passes through the separation membrane 12 and flows into the second flow paths 16. Water vapor that has flowed into the second flow paths 16 is taken into sweep gas flowing into the second flow paths 16 from the sweep gas inlet T3 via the second permeate-side space S22 and the second slits 18, and thereafter is discharged from the sweep gas outlet T4 to the outside via the first slits 17 and the first permeate-side space S21.

Note that, in this variation, the sweep gas inlet T3 and the sweep gas outlet T4 are provided on straight lines intersecting the axis of the reactor 10 in the cross-sectional view. With this configuration, the length of a flow path of the sweep gas in the first permeate-side space S21 and the length of a flow path of the sweep gas in the second permeate-side space S22 can be made equivalent to each other, and accordingly, it is possible to suppress maldistribution of flows of the sweep gas. However, the positional relationship between the sweep gas inlet T3 and the sweep gas outlet T4 can be changed as appropriate.

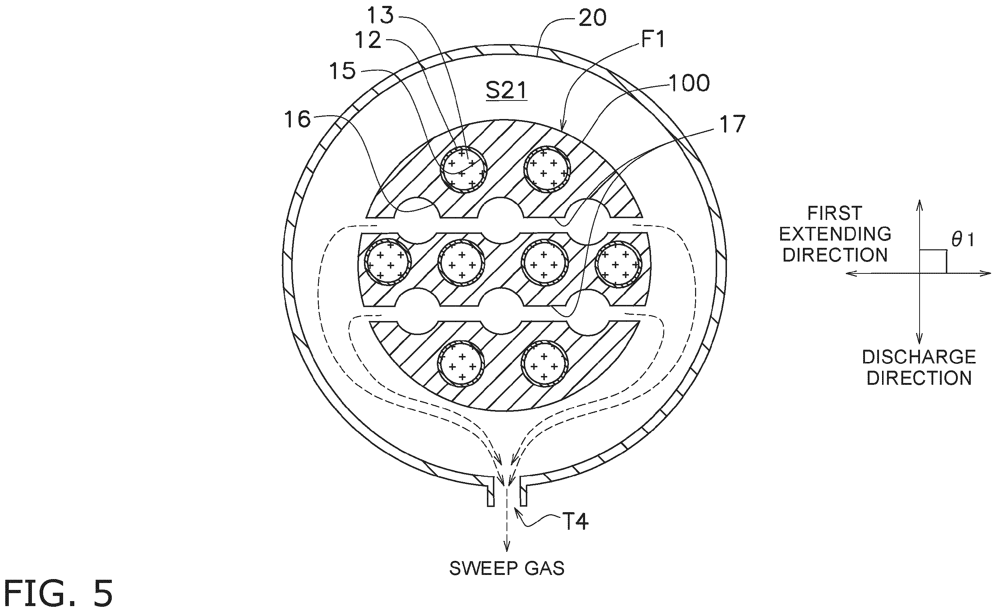

FIG. 5 is a cross-sectional view taken along a line A-A shown in FIG. 4. As shown in FIG. 5, the first slits 17 linearly extend through the inside of the reactor 100 and are open on both sides of the reactor 100. Each first slit 17 includes two openings formed in the outer circumferential surface F1. Sweep gas that has flowed out from the two openings of the first slit 17 to the first permeate-side space S21 passes through the first permeate-side space S21 and is discharged from the sweep gas outlet T4 to the outside.

Here, a first extending direction of each first slit 17 extending inside the reactor 100 is preferably inclined with respect to a discharge direction of the sweep gas discharged from the sweep gas outlet T4 to the outside, or the first extending direction is preferably orthogonal to the discharge direction. Specifically, an angle θ1 of the first extending direction relative to the discharge direction is preferably 45° or more and 135° or less. With this configuration, it is possible to suppress maldistribution of gas flows from the openings of the first slits 17 toward the sweep gas outlet T4, and accordingly, it is possible to suppress maldistribution of flows of sweep gas in the first permeate-side space S21.

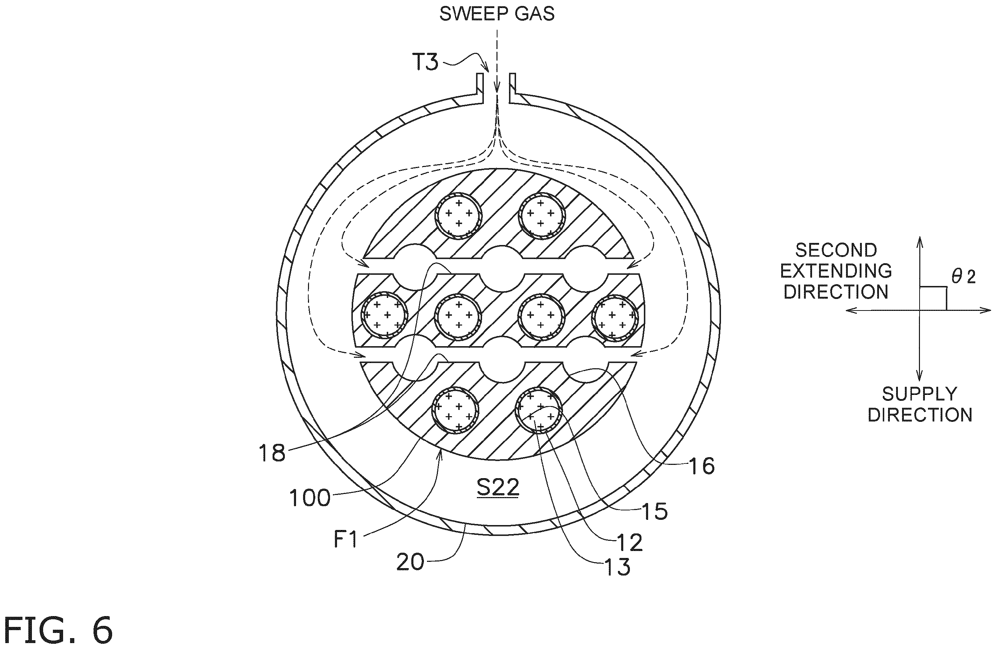

FIG. 6 is a cross-sectional view taken along a line B-B shown in FIG. 4. As shown in FIG. 6, the second slits 18 linearly extend through the inside of the reactor 100 and are open on both sides of the reactor 100. Each second slit 18 includes two openings formed in the outer circumferential surface F1. Sweep gas supplied from the sweep gas inlet T3 to the second permeate-side space S22 passes through the second permeate-side space S22 and flows into the two openings of the second slit 18.

Here, a second extending direction of each second slit 18 extending inside the reactor 100 is preferably inclined with respect to a supply direction of the sweep gas supplied from the sweep gas inlet T3 to the second permeate-side space S22, or the second extending direction is preferably orthogonal to the supply direction. Specifically, an angle θ2 of the second extending direction relative to the supply direction is preferably 45° or more and 135° or less. With this configuration, it is possible to suppress maldistribution of gas flows from the sweep gas inlet T3 toward the openings of the second slits 18, and accordingly, it is possible to suppress maldistribution of flows of sweep gas in the second permeate-side space S22.

### [Variation 3]

In the above embodiment, the separation membrane 12 is in contact with the catalyst 13, but a buffer layer may be interposed between the separation membrane 12 and the catalyst 13. The buffer layer physically separates the catalyst 13 from the separation membrane 12, and therefore, when the catalyst 13 has a high temperature due to reaction heat, it is possible to suppress formation of a crack in the separation membrane 12 from a point that is in contact with the catalyst 13. The buffer layer can be constituted by a ceramic material or an organic polymer material. Examples of ceramic materials that can be used include silica, alumina, and chromium oxide. Examples of organic polymer materials that can be used include PTFE, PVA, and PEG.

### [Variation 4]

In the above embodiment, the separation membrane 12 allows permeation of water vapor, which is a product of the conversion reaction for converting the source gas into the liquid fuel, but there is no limitation to this configuration. The separation membrane 12 may allow permeation of the liquid fuel generated through the conversion reaction for converting the source gas into the liquid fuel. In this case as well, the reaction equilibrium of the above formula (1) can be shifted to the product side.

Also, in the case where the separation membrane 12 allows permeation of the liquid fuel, the reaction equilibrium can be shifted to the product side even when the liquid fuel is generated through a reaction in which no water vapor is generated as a by-product (e.g., H₂+CO ⇔ CH₃OH).

### [Variation 5]

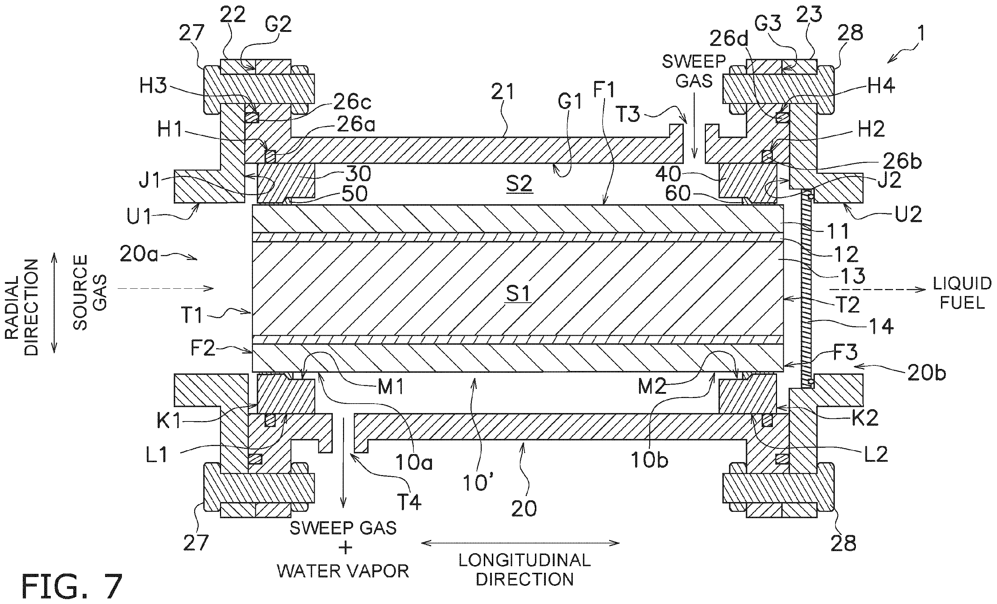

In the above embodiment, a portion of the reactor 10 is inserted into the first opening 20a, but when the longitudinal dimension of the reactor 10 is short as shown in FIG. 7, the reactor 10 may not be inserted into the first opening 20a.

Likewise, in the above embodiment, a portion of the reactor 10 is inserted into the second opening 20b, but when the longitudinal dimension of the reactor 10 is short as shown in FIG. 7, the reactor 10 may not be inserted into the second opening 20b.

### [Variation 6]

In the separation membrane module 1 (see FIG. 1) according to the above embodiment, the sweep gas inlet T3 and the sweep gas outlet T4 are diagonally opposite to each other in a side view, and sweep gas flows from the sweep gas inlet T3 toward the sweep gas outlet T4, but there is no limitation to this configuration.

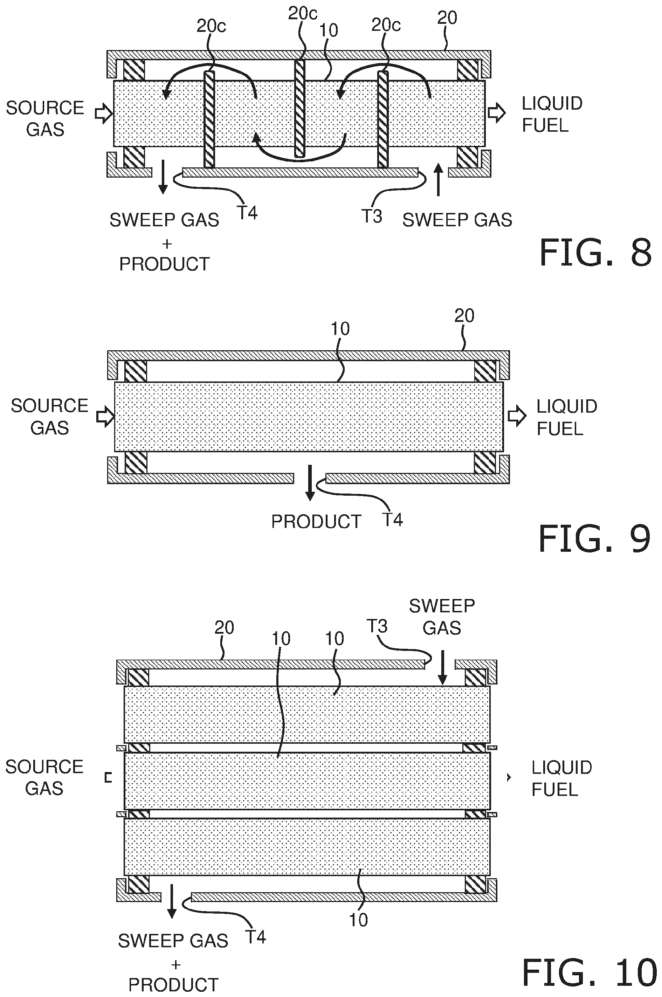

For example, as shown in FIG. 8, the sweep gas inlet T3 and the sweep gas outlet T4 may be provided on the same side of the reactor 10 in a side view, and flow regulation plates 20c that partially block the space between the reactor 10 and the housing 20 may be provided. Three flow regulation plates 20c are provided in the example shown in FIG. 8, but the number of flow regulation plates 20c may be one, two, or four or more. Positions of the flow regulation plates 20c can be suitably set.

As shown in FIG. 9, a configuration is also possible in which the housing 20 does not have the sweep gas inlet T3, and sweep gas is not supplied into the housing 20. In this case, a product separated by the separation membrane 12 of the reactor 10 flows out from the sweep gas outlet T4 to the outside of the housing 20. The position of the sweep gas outlet T4 can be suitably set.

Note that the configurations shown in FIGS. 8 and 9 are also applicable to the case where a separation filter is used as the membrane structure instead of the reactor 10.

### [Variation 7]

In the separation membrane module 1 (see FIG. 1) according to the above embodiment, only one reactor 10 is housed in the housing 20, but there is no limitation to this configuration.

For example, a plurality of reactors 10 may be housed in the housing 20 as shown in FIG. 10.

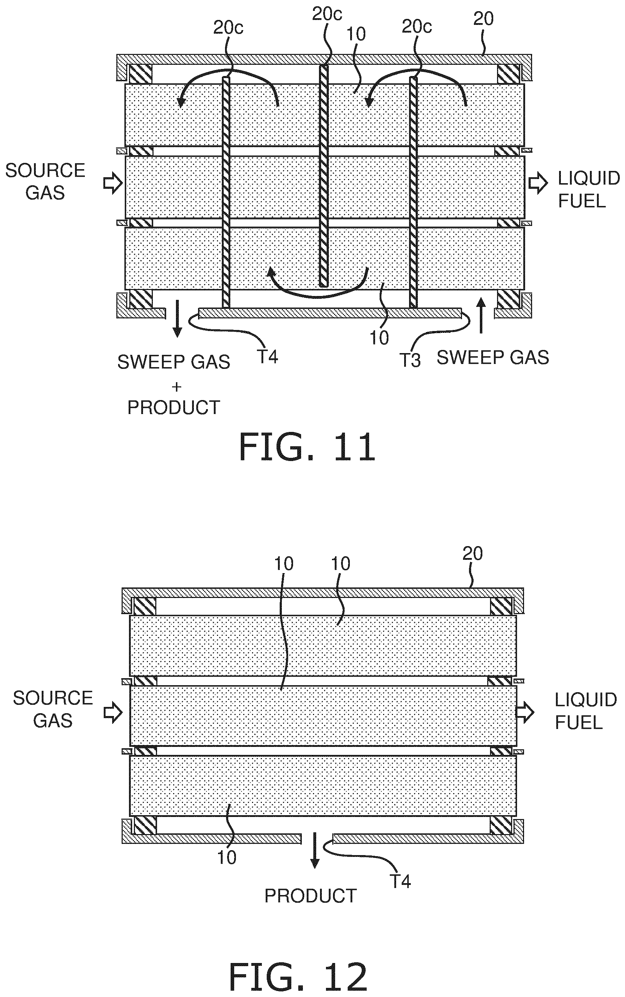

Also, flow regulation plates 20c that partially block the space between the reactors 10 and the housing 20 may be provided as shown in FIG. 11. Three flow regulation plates 20c are provided in the example shown in FIG. 11, but the number of flow regulation plates 20c may be one, two, or four or more. Positions of the flow regulation plates 20c can be suitably set. Note that the sweep gas inlet T3 and the sweep gas outlet T4 may be provided on the same side of the reactors 10 in a side view as shown in FIG. 11.

As shown in FIG. 12, a configuration is also possible in which the housing 20 does not have the sweep gas inlet T3, and sweep gas is not supplied into the housing 20. In this case, a product separated by the separation membrane 12 of the reactors 10 flows out from the sweep gas outlet T4 to the outside of the housing 20. The position of the sweep gas outlet T4 can be suitably set.

Note that the configurations shown in FIGS. 10 to 12 are also applicable to a case where separation filters are used as the membrane structures instead of the reactors 10.

### [Variation 8]

In the separation membrane module 1a (see FIG. 4) according to Variation 2 described above, the sweep gas inlet T3 and the sweep gas outlet T4 are diagonally opposite to each other in a side view, but there is no limitation to this configuration.

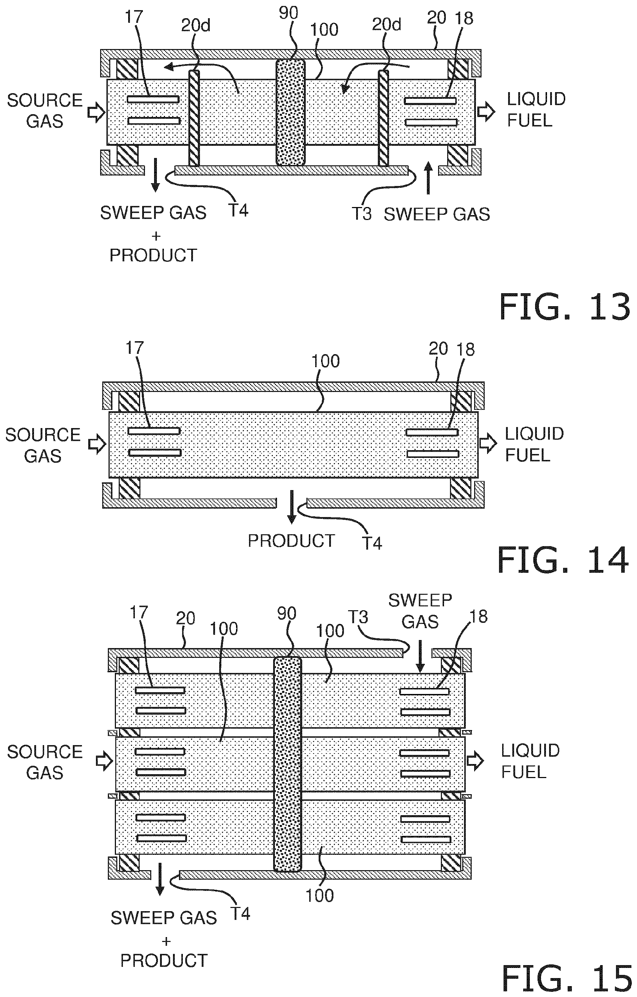

For example, as shown in FIG. 13, the sweep gas inlet T3 and the sweep gas outlet T4 may be provided on the same side of the reactor 100 in a side view, and flow regulation plates 20d that partially block the space between the reactor 100 and the housing 20 may be provided. In the example shown in FIG. 13, a flow regulation plate 20d is provided on each side of the flow stopper 90. The sweep gas inlet T3-side flow regulation plate 20d divides the sweep gas inlet T3-side space into a space in which sweep gas mainly flows into the second slits 18 in the reactor 100 and a space in which sweep gas flows into the reactor 100 from its side surface. The sweep gas outlet T4-side flow regulation plate 20d divides the sweep gas outlet T4-side space into a space in which sweep gas mainly flows out from the first slits 17 in the reactor 100 and a space in which sweep gas flows out from the side surface of the reactor 100. However, the number and positions of flow regulation plates 20d can be suitably set. Also, a portion of sweep gas may pass through the flow stopper 90.

As shown in FIG. 14, a configuration is also possible in which the housing 20 does not have the sweep gas inlet T3, and sweep gas is not supplied into the housing 20. In this case, a product separated by the separation membrane 12 of the reactor 100 flows out from the sweep gas outlet T4 to the outside of the housing 20. The position of the sweep gas outlet T4 can be suitably set.

Note that the configurations shown in FIGS. 13 and 14 are also applicable to a case where a separation filter is used as the membrane structure instead of the reactor 100.

### [Variation 9]

In the separation membrane module 1a (see FIG. 4) according to Variation 2 described above, only one reactor 100 is housed in the housing 20, but there is no limitation to this configuration.

For example, a plurality of reactors 100 may be housed in the housing 20 as shown in FIG. 15.

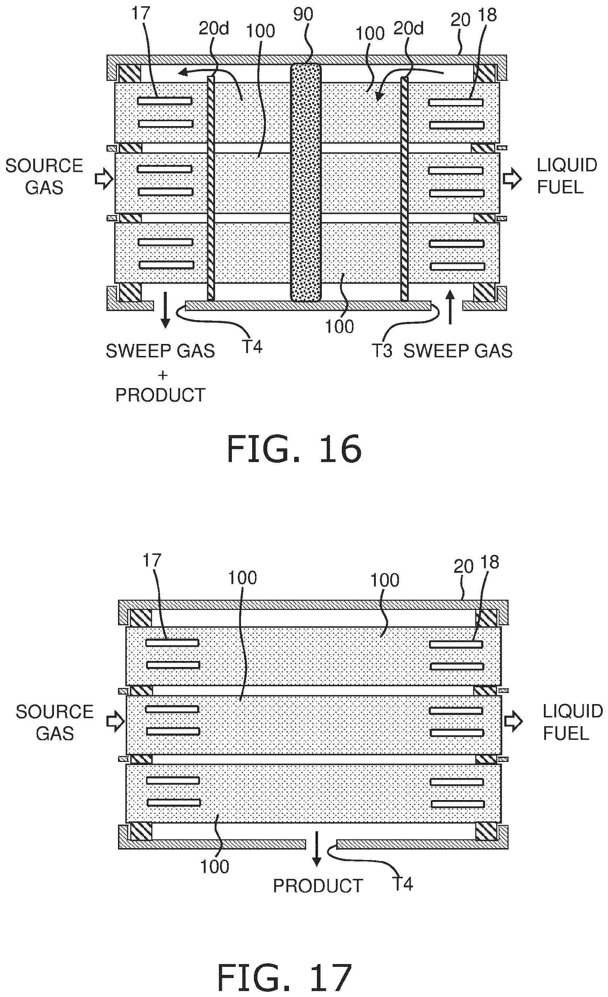

Also, flow regulation plates 20d that partially block the space between the reactors 100 and the housing 20 may be provided as shown in FIG. 16. In the example shown in FIG. 16, a flow regulation plate 20d is provided on each side of the flow stopper 90. The sweep gas inlet T3-side flow regulation plate 20d divides the sweep gas inlet T3-side space into a space in which sweep gas mainly flows into the second slits 18 in the reactors 100 and a space in which sweep gas flows into the reactors 100 from their side surfaces. The sweep gas outlet T4-side flow regulation plate 20d divides the sweep gas outlet T4-side space into a space in which sweep gas mainly flows out from the first slits 17 in the reactors 100 and a space in which sweep gas flows out from the side surfaces of the reactors 100. However, the number and positions of flow regulation plates 20d can be suitably set. Also, a portion of sweep gas may pass through the flow stopper 90. Note that the sweep gas inlet T3 and the sweep gas outlet T4 may be provided on the same side of the reactors 100 in a side view as shown in FIG. 16.

As shown in FIG. 17, a configuration is also possible in which the housing 20 does not have the sweep gas inlet T3, and sweep gas is not supplied into the housing 20. In this case, a product separated by the separation membrane 12 of the reactors 100 flows out from the sweep gas outlet T4 to the outside of the housing 20. The position of the sweep gas outlet T4 can be suitably set.

Note that the configurations shown in FIGS. 15 to 17 are also applicable to a case where separation filters are used as the membrane structures instead of the reactors 100.

### REFERENCE SIGNS LIST

- 1: Separation membrane module
- 10: Reactor
- 10a: First end portion
- 10b: Second end portion
- F1: Outer circumferential surface
- F2: First end surface
- F3: Second end surface
- 20: Housing
- 20a: First opening
- U1: Inner circumferential surface
- 20b: Second opening
- U2: Inner circumferential surface
- 21: Tube main body
- 22: First end plate
- 23: Second end plate
- 30: First flange
- K1: End surface
- 40: Second flange
- K2: End surface
- 50: First joining material
- 60: Second joining material

## Claims

1. A separation membrane module comprising:
a tubular housing;
a columnar membrane structure housed in the housing and extending in a longitudinal direction; and
an annular first flange surrounding a first end portion of the membrane structure; wherein
a first end surface of the membrane structure is located outward of an end surface of the first flange in the longitudinal direction.

2. The separation membrane module according to claim 1, wherein
the housing includes a first opening having a center on an axis of the membrane structure, and
an outer circumferential surface of the membrane structure is located inward of an inner circumferential surface of the first opening in a radial direction perpendicular to the longitudinal direction.

3. The separation membrane module according to claim 2, wherein
a portion of the membrane structure is inserted into the first opening.

4. The separation membrane module according to claim 1, further comprising:
a first joining material interposed between the membrane structure and the first flange, wherein
the first joining material is spaced apart from the housing.

5. The separation membrane module according to claim 1, wherein
the first flange is constituted by a ceramic material.

6. The separation membrane module according to any one of claims 1 to 5, further comprising:
an annular second flange surrounding a second end portion of the membrane structure, wherein
a second end surface of the membrane structure is located outward of an end surface of the second flange in the longitudinal direction.

7. The separation membrane module according to claim 6, wherein
the housing includes a second opening having a center on an axis of the membrane structure, and
an outer circumferential surface of the membrane structure is located inward of an inner circumferential surface of the second opening in the radial direction.

8. The separation membrane module according to claim 7, wherein
a portion of the membrane structure is inserted into the second opening.

9. The separation membrane module according to claim 6, further comprising:
a second joining material interposed between the membrane structure and the second flange, wherein
the second joining material is spaced apart from the housing.

10. The separation membrane module according to claim 6, wherein
the second flange is constituted by a ceramic material.

11. The separation membrane module according to claim 1, wherein
the membrane structure is a reactor.

12. The separation membrane module according to claim 1, wherein
the membrane structure is a separation filter.
